Europäisches Patentamt

European Patent Office

Office européen des brevets

(11) Veröffentlichungsnummer: **0 226 860**
B1

(12) **EUROPÄISCHE PATENTSCHRIFT**

(45) Veröffentlichungstag der Patentschrift:
03.01.90

(51) Int. Cl.⁴: **A61K 7/09**, A61K 7/13

(21) Anmeldenummer: **86116445.7**

(22) Anmeldetag: **26.11.86**

(54) **Verfahren zum gleichzeitigen Färben und dauerhaften Verformen von Haaren.**

(30) Priorität: **09.12.85  DE 3543453**

(43) Veröffentlichungstag der Anmeldung:
**01.07.87 Patentblatt 87/27**

(45) Bekanntmachung des Hinweises auf die Patenterteilung:
**03.01.90 Patentblatt 90/1**

(84) Benannte Vertragsstaaten:
**AT BE CH DE ES FR GB GR IT LI LU NL SE**

(56) Entgegenhaltungen:
**FR-A- 2 551 973**
**GB-A- 876 663**
**GB-A- 1 077 758**
**US-A- 3 396 736**

(73) Patentinhaber: **Wella Aktiengesellschaft, Berliner Allee 65, D-6100 Darmstadt(DE)**

(72) Erfinder: **Wajaroff, Theodor, Erbacher Strasse 56 B, D-6100 Darmstadt(DE)**
Erfinder: **Hartmann, Peter, Hoffmannstrasse 6, D-6100 Darmstadt(DE)**
Erfinder: **Kubo, Kohei, 4-26-29 Kitazawa, Setagaya-ku Tokio(JP)**

## Beschreibung

Gegenstand der Erfindung ist ein Verfahren zur dauerhaften Verformung von Weißem und grauem Haar, welches eine gleichzeitige Färbung der Haare bewirkt.

Die dauerhafte Haarverformung erfolgt bei den zur Zeit üblichen Verfahren in zwei Stufen. Zunächst werden durch die Einwirkung eines geeigneten Reduktionsmittels die Disulfidbrücken des Haarkeratins gespalten. Das Haar wird sodann in seine neue Form gebracht und anschließend durch die Behandlung mit einem geeigneten Oxidationsmittel, unter Wiederverknüpfung der gespaltenen Disulfidbindungen, in der neuen Form fixiert.

Die zur Durchführung der ersten, reduzierenden Verfahrensstufe verwendeten Mittel enthalten als verformungswirksame, haarkeratinreduzierende Substanz Sulfit, Bisulfit oder bestimmte Mercaptoverbindungen, insbesondere Thioglykolsäure und Thiomilchsäure, auch in Form ihrer Ester oder Alkali- oder Ammoniumsalze.

Diese Mittel sind entweder sauer (Sulfit, Bisulfit und Mercaptocarbonsäureester) oder alkalisch (Alkali- und Ammoniumsalze von Mercaptocarbonsäuren) eingestellt. Im Falle von alkalisch eingestellten Verformungsmitteln wird die erforderliche Alkalität vor allem durch Zusatz von Ammoniak, organischen Aminen, Ammonium- oder Alkalicarbonat und Ammonium- oder Alkalihydrogencarbonat erreicht.

Die Durchführung der dauerhaften Verformung von menschlichen Haaren erfolgt im allgemeinen, indem man das gewaschene und handtuchtrockene Haar zunächst in mehrere Partien aufteilt und diese Partien sodann auf Wickler wickelt. Nach Beendigung des Wickelvorganges werden die Wickler mit der erforderlichen Menge des Dauerverformungsmittels gründlich durchfeuchtet. Die für eine Dauerwellung verwendeten Wickler haben einen Durchmesser von etwa 5 bis 13 Millimetern, während für eine Haarentkräuselung Wickler mit einem Durchmesser von über 13 Millimetern erforderlich sind. Bei der Haarentkräuselung kann auf die Verwendung von Wicklern auch verzichtet werden, wenn das Haar durch Kämmen während der Einwirkungszeit des keratinreduzierenden Mittels gestreckt wird.

Die Einwirkungszeit des Verformungsmittels auf das Haar beträgt, sowohl bei der Dauerwellung als auch bei der dauerhaften Entkräuselung, je nach Haarbeschaffenheit und dem gewünschten Grad der Umformung etwa 5 bis 30 Minuten. Durch Wärmezufuhr, beispielsweise unter Verwendung eines Wärmestrahlers oder einer Trockenhaube, läßt sich diese Einwirkungszeit verkürzen.

Nach Ablauf der erforderlichen Einwirkungszeit des Verformungsmittels wird das Haar mit Wasser gespült und mit einem Fixiermittel, zum Beispiel einer wäßrigen Lösung von Hydrogenperoxid oder Kaliumbromat, behandelt. Die Einwirkungszeit des Fixiermittels beträgt hierbei üblicherweise etwa 10 bis 15 Minuten. Anschließend werden die Wickler entfernt, gegebenenfalls das Haar nochmals einige Minuten mit dem Fixiermittel nachbehandelt und sodann gründlich mit Wasser ausgespült, zur Frisur gelegt und getrocknet.

Häufig, insbesondere bei weißem oder grauem Haar, wird aber nicht nur eine Haarverformung, sondern zusätzlich auch eine Färbung oder Tönung des Haares gewünscht. Hierbei ist es dann erforderlich, vor oder nach der Verformungsbehandlung eine gesonderte Färbebehandlung durchzuführen. Dies führt zu einer übermäßigen Beanspruchung des Haares, da jede Färbung oder Haarverformung einen schwerwiegenden Eingriff in die Haarstruktur darstellt.

Eine Verringerung dieser Beanspruchung der Harre kann durch eine gleichzeitige Durchführung von Haarverformungs- und Färbebehandlung erreicht werden. Eine derartige Kombination der beiden Behandlungen ist zudem auch zeitsparend.

Aus diesem Grunde wurde bereits mehrfach versucht, die Verformung und Färbung von Haaren in einem Arbeitsgang durchzuführen. So ist zum Beispiel aus der DE-AS 1 129 261 sowie der GB-PS 876 663 ein Verfahren bekannt, das eine gleichzeitige Dauerwellung und Färbung von Haaren, unter anderem auch von weißem und grauem Haar, ermöglicht. Dieses Verfahren basiert auf einem Verformungsmittel, welches aus einer wäßrigen Lösung eines keratinreduzierenden Wirkstoffes und eines geeigneten basischen Farbstoffes besteht. Die verwendeten Farbstoffe, wie zum Beispiel Kristallviolett, Methylenblau, Fuchsin oder Malachitgrün, liegen in Form der stabileren Leukoverbindungen vor und werden erst bei der anschließenden oxidativen Fixierung in den eigentlichen Farbstoff umgewandelt. Weiterhin wird in der deutschen Patentanmeldung A 16 016 sowie der GB-PS 721 831 vorgeschlagen, bestimmte saure wasserlösliche Azofarbstoffe, wie zum Beispiel Resorcinbraun, Orange I und Tartrazin, den Haarverformungsmitteln zuzusetzen, um so gleichzeitig mit der Verformungsbehandlung eine Haartönung zu erreichen. Für den gleichen Zweck werden in der FR-PS 1 129 112 Nitroverbindungen der allgemeinen Formel $(NO_2)_x RX_Y(NHR')_z$ empfohlen, wobei R eine aromatische Gruppe bedeutet, X gleich einer Amino- oder Hydroxygruppe ist, und R' einen aliphatischen Rest mit mindestens einer Hydroxygruppe darstellt. Abschließend sei auf den Übersichtsartikel "Dauerwellen und Haarfärben in einem Arbeitsgang" von R. Heilingötter, Kosmetik-Parfüm-Drogen Rundschau 3/4 (1965), Seiten 35 und 36, hingewiesen, in dem die Möglichkeit einer gleichzeitigen Tönung und Verformung der Haare durch Zusatz von Oxidationsfarbstoffen (in Form ihrer Vorstufen) zu einer alkalischen Thioglykolatlösung diskutiert wird.

Alle bisher beschriebenen Verfahren zur dauerhaften Verformung und gleichzeitigen Färbung oder Tönung der Haare weisen jedoch Nachteile auf, so daß die erzielten Ergebnisse nicht immer zufriedenstellend sind. So sind einige der verwendeten Farbstoffe, wie beispielsweise bestimmte Oxidationshaarfarbstoffe oder die in der FR-PS 1 129 112 beschriebenen Nitrofarbstoffe, gegenüber der stark reduzierend wirkenden, alkalischen Thioglykolatlösung unbeständig. Sie werden bei längerer Lagerung reduktiv angegriffen und lassen in ihrer Färbekraft nach, so daß die Herstellung gebrauchsfertiger Zubereitungen unmöglich ist. Weiterhin besitzen die in der FR-PS 1 129 112 verwendeten Nitro farbstoffe den Nachteil, daß sie nur gelbe, orange und rote Farbtöne umfassen. Die für weißes und graues Haar besonders benötigten Blau- und Violett-Töne sind mit diesen Nitrofarbstoffen nicht erhältlich. Andere zur gleichzeitigen Haarfärbung bei ver Verformungsbehandlung verwendeten Farbstoffe, wie beispielsweise die in der DE-AS 1 129 261 und der GB-PS 876 663 beschriebenen Farbstoffe, besitzen nur eine mäßige Lichtechtheit.

Aufgabe der vorliegenden Erfindung ist es daher, ein Verfahren zur Verfügung zu stellen, das es besser als bisher ermöglicht, graues oder weißes Haar bei der Verformungsbehandlung gleichzeitig zu färben und so den unerwünschten gelben Ton des Haares zu entfernen.

Es wurde nun überraschenderweise gefunden, daß der störende Gelbton bei weißem oder grauem Haar während der Verformungsbehandlung in besonders vorteilhafter Weise dauerhaft beseitigt wird, wenn dem Verformungsmittel bestimmte rotviolette Farbstoffe und dem Fixiermittel bestimmte blaue Farbstoffe zugesetzt werden.

Gegenstand der vorliegenden Erfindung ist daher ein Verfahren zum gleichzeitigen Färben und dauerhaften Verformen von weißem und grauem Haar, bei dem man das Haar zuerst mit einem Verformungsmittel behandelt, mit Wasser spült und sodann mit einem Fixiermittel behandelt, welches dadurch gekennzeichnet ist, daß man ein Verformungsmittel mit einem Gehalt an mindestens einem rotviolett-färbenden Xanthenfarbstoff und ein Fixiermittel mit einem Gehalt an mindestens einem blaufärbenden Anthrachinonfarbstoff verwendet.

Bei dem erfindungsgemäßen Verfahren wird weißes oder graues Haar gewaschen, mit einem Handtuch frottiert, gegebenenfalls mit einem Teil des Haarverformungsmittels vorgefeuchtet, in einzelne Strähnen aufgeteilt und auf Wickler gewickelt. Der Durchmesser der Wickler beträgt hierbei, je nachdem ob eine Dauerwellung oder eine Haarentkräuselung gewünscht wird, entweder etwa 5 bis 13 Millimeter oder etwa 15 bis 35 Millimeter. Auf das aufgewickelte Haar wird anschließend eine für die Haarverformung ausreichende Menge des Verformungsmittels, im allgemeinen etwa 80 g, aufgetragen. Die bei dem hier beschriebenen Verfahren verwendbaren Verformungsmittel sind solche auf der Basis üblicher haarkeratinreduzierender Stoffe, wie zum Beispiel Salze der schwefeligen Säure oder bestimmte Mercaptoverbindungen, insbesondere Salze oder Ester von Mercaptocarbonsäuren. Diese Verformungsmittel enthalten die reduzierenden Verbindungen in den für die Haarverformung üblichen Mengen, beispielsweise die Ammoniumsalze der Thioglykol- oder Thiomilchsäure, in einer Konzentration von etwa 2 bis 12 Gewichtsprozent. Der pH-Wert der alkalischen Verformungsmittel liegt im allgemeinen bei 7 bis 10, wobei die Einstellung vorzugsweise mit Ammoniak, Monoethanolamin, Ammoniumcarbonat oder Ammoniumhydrogencarbonat erfolgt. Bei sauer (zum Beispiel auf pH = 6,5 bis 6,9) eingestellten Verformungsmitteln werden vorzugsweise Ester von Mercaptocarbonsäuren, wie beispielsweise Monothioglykolsäureglykolester oder -glycerinester, in einer Konzentration von 2 bis 14 Gewichtsprozent sowie Salze der schwefeligen Säure, zum Beispiel Natrium-, Ammonium- oder Monoethanolammoniumsulfit, in einer Konzentration von 3 bis 8 Gewichtsprozent (berechnet als $SO_2$) verwendet.

Zur Wirkungssteigerung können diesen Verformungsmitteln Quell- und Penetrationsstoffe, wie beispielsweise Harnstoff, Melamin, Alkali- oder Ammoniumthiocyanat, Isopropanol, Imidazolidin-2-on, 2-Pyrrolidon und 1-Methyl-2-pyrrolidon, in einer Konzentration von etwa 0,5 bis 50 Gewichtsprozent, vorzugsweise 2 bis 30 Gewichtsprozent, zugesetzt werden.

Zusätzlich enthalten die bei dem erfindungsgemäßen Verfahren verwendeten Verformungsmittel mindestens einen rotviolett-färbenden Xanthenfarbstoff in einer Konzentration von 0,00002 bis 0,012 Gewichtsprozent, vorzugsweise 0,0001 bis 0,005 Gewichtsprozent.

Dieser Xanthenfarbstoff ist ausgewählt aus im Colour-Index, Volume 4, Third Edition, The Society of Dyers and Colourists, Bradford and London 1971, Seite 4417 ff, aufgeführten Farbstoffen mit den folgenden Colour-Index-Nummern: C. I. 45 410 (D & C Red. No. 27 und 28, Acid Red 218, C. I. Solvent Red 48, C. I. Acid Red 92, C. I. Pigment Red 174), C. I. 45 190 (Ext. D & C Red No. 3, Red 401, C. I. Acid Violet 9, C. I. Solvent Violet 10) und C. I. 45 170 (D & C Red No. 19 und 37, C-ext. Rot 27, C. J. Basic Violet 10, C. I. Solvent Red 49, C. I. Pigment Red 173, C. I. Pigment Violet 1, Red 213, Red 215).

Nach einer für die dauerhafte Verformung des Haares ausreichenden Einwirkungszeit, welche je nach Haarbeschaffenheit, dem pH-Wert und der Verformungswirksamkeit des Verformungsmittels sowie in Abhängigkeit von der Anwendungstemperatur etwa 5 bis 30 Minuten beträgt, wird das Verformungsmittel mit Wasser ausgespült und das Haar oxidativ fixiert. Das Fixiermittel wird, je nach Haarfülle, in einer Menge von etwa 80 bis 100 g verwendet.

Für die Fixierung kann jedes beliebige, bisher in Fixiermitteln verwendete Oxidationsmittel angewendet werden. Beispiele für solche Oxidationsmittel sind Kaliumbromat, Natriumbromat, Natriumperborat, Hydrogenperoxid und Harnstoffperoxid. Die Konzentration der Oxidationsmittel ist in Abhängigkeit von Anwendungszeit (in der Regel 5 bis 15 Minuten) und Anwendungstemperatur unterschiedlich. Normalerweise werden in den wäßrigen Fixiermitteln die Oxidationsmittel in einer Konzentration von etwa 0,5 bis 10,0 Gewichtsprozent verwendet. Das Fixiermittel kann selbstverständlich weitere Stoffe, wie zum Beispiel schwache Säuren oder Peroxidstabilisatoren, enthalten.

Zusätzlich enthalten diese Fixiermittel mindestens einen der im Colour-Index, Volume 4, Third Edition, The Society of Dyers and Colourists, Bradford and London 1971, Seite 4511 ff., unter den folgenden Colour-Index Nummern beschriebenen Anthrachinonfarbstoffe: C. I. 69 825 (D & C Blue No. 9, Blue 204, C. I. Vat Blue 6, C. I. Pigment Blue 64), C. I. 60 725 (D & C Violet No. 2, Violet 201, C. I. Solvent Violet 13) und C. I. 60 730 (Ext. D & C Violet No. 2, Violet 401, C. I. Acid Violet 43).

Der Gehalt an diesen Anthrachinonfarbstoffen in den Fixiermitteln beträgt 0,00002 bis 0,012 Gewichtsprozent, vorzugsweise 0,0001 bis 0,01 Gewichtsprozent.

Sowohl die bei dem erfindungsgemäßen Vefahren verwendeten Verformungs mittel als auch die Fixiermittel können in Form einer wäßrigen Lösung oder einer Emulsion sowie in verdickter Form auf wäßriger Basis, insbesondere als Creme, Gel oder Paste, vorliegen. Ebenfalls ist es möglich, diese Mittel unter Druck in Aerosoldosen abzufüllen und daraus als Aerosolschaum zu entnehmen.

Selbstverständlich können sowohl die Fixiermittel als auch die Verformungsmittel alle für derartige Mittel üblichen und bekannten Zusatzstoffe, zum Beispiel Verdickungsmittel, wie beispielsweise Kaolin, Bentonit, Fettsäuren, höhere Fettalkohole, Stärke, Polyacrylsäure, Cellulosederivate, Alginate, Vaseline oder Paraffinöl, Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quartäre Ammoniumsalze, Alkylbetaine, oxethylierte Fettalkohole, oxethylierte Alkylphenole,Fettsäurealkanolamide oder oxethylierte Fettsäurester, ferner Trübungsmittel, wie zum Beispiel Polyethylenglykolester, oder Alkohole, wie beispielsweise Ethanol, Propanol, Isopropanol oder Glycerin, Lösungsvermittler, Stabilisatoren, Puffersubstanzen, Parfümöle, Haarkonditionierungsmittel sowie haarpflegende Bestandteile, wie zum Beispiel Lanolinderivate, Cholesterin, Pantothensäure oder Betain, enthalten. Ferner können diesen Mitteln optische Aufheller in Form von Cumarin-, Stilben-, Naphthalimid-, Benzoxazol- oder Styrylderivaten zugesetzt werden. Die erwähnten Bestandteile werden in den für solche Zwecke üblichen Mengen verwendet, zum Beispiel die Netzmittel und Emulgatoren in Konzentrationen von etwa 0,2 bis 30 Gewichtsprozent, während die Verdickungsmittel in einer Menge von etwa 0,1 bis 25 Gewichtsprozent in diesen Mitteln enthalten sein können.

Anschließend werden die Wickler entfernt, das Fixiermittel mit Wasser aus dem Haar ausgespült und das Haar in üblicher Weise weiterbehandelt. In der Regel wird das Haar im Anschluß an eine Dauerverformung zur Wasserwelle gelegt. Im Falle einer Haarentkräuselung kann auch so verfahren werden, daß man das Fixiermittel bei gewickeltem Haar abspült und das Haar anschließend, ohne abzuwickeln, direkt am Wickler trocknet. Weiterhin ist es auch möglich, das entkräuselte Haar nach dem Entfernen der Wickler und dem Abspülen des Fixiermittels unmittelbar trocken zu fönen.

Das auf diese Weise dauerverformte Haar besitzt eine ansprechende, klare, leuchtend weiße Farbe, welche frei von Gelbtönen ist.

Das erfindungsgemäße Verfahren bietet den Vorteil, daß eine Verformung des Haares und eine Beseitigung des bei weißem und grauem Haar häufig auftretenden, unerwünschten Gelbtons in einem Arbeitsgang erreicht wird. Hierdurch wird eine schonende und zugleich zeitsparende Behandlung des Haares ermöglicht.

Ein weiterer Vorteil der vorliegenden Erfindung ist die einfache Unterscheidung von Verformungsmitteln und Fixiermittel anhand der unterschiedlichen Farbe dieser beiden Mittel (Fixiermittel = blau; Verformungsmittel = rotviolett), wodurch eine sichere Durchführung des Verfahrens gewährleistet wird.

Die folgenden Beispiele dienen der näheren Erläuterung der Erfindung.

<u>Beispiele</u>

<u>Beispiel 1</u> Verfahren zur Dauerwellung und gleichzeitigen Färbung von weißen und grauem Haar

Zu 50 Prozent ergrautes, weißes Haar wird mit einem milden Shampoo gewaschen. Anschließend wird das handtuchtrockene Haar auf Wickler mit einem Durchmesser von 7 bis 10 Millimetern gewickelt und mit einer Dauerwellflüssigkeit der folgenden Zusammensetzung

| | |
|---|---|
| 23,700 g | Ammoniumthioglykolat, 50prozentige wäßrige Lösung |
| 3,000 g | Ammoniumcarbonat |
| 5,000 g | Ammoniumhydrogencarbonat |
| 2,000 g | Isooctylphenol, mit 10 Mol Ethylenoxid oxethyliert |
| 0,200 g | Parfümöl |
| 0,001 g | C.I. Acid Violet 9 (C.I. 45 190) |
| 66,099 g | Wasser |
| 100,000 g | |

mehrmals gut durchfeuchtet. Der pH-Wert der verwendeten Dauerwellflüssigkeit ist 8,8.

Nach einer Einwirkungszeit von 15 Minuten wird das gewickelte Haar gründlich mit warmem Wasser gespült und anschließend mit einem Fixiermittel der folgenden Zusammensetzung

| | |
|---|---|
| 9,200 g | Natriumbromat |
| 1,000 g | 1-Carboxymethyl-2-heptadecyl-1-(2-hydroxyethyl)-2-imidazoliniumchlorid (Miranol®DM) |
| 1,000 g | Isooctylphenol, mit 10 Mol Ethylenoxid oxethyliert |
| 0,100 g | Parfümöl |
| 0,002 g | C.I. Vat Blue 6 (C.I. 69 825) |
| 88,698 g | Wasser |
| 100,000 g | |

und einem pH-Wert von 6,8 behandelt.

Nach einer Einwirkungszeit von 5 Minuten werden die gewickelten Haare mit Wasser gespült und die Wickler aus den Haaren entfernt. Anschließend wird das Haar zur Wasserwelle gelegt und unter einer Trockenhaube getrocknet.

Als Ergebnis dieser Behandlung wird ein dauergewelltes Haar erhalten, dessen Weißanteil eine strahlend weiße, von Gelbtönen freie, Farbe besitzt. Diese Färbung ist sehr beständig und bleibt auch nach mehrmaliger Haarwäsche erhalten.

Beispiel 2 Verfahren zur dauerhaften Verformung und gleichzeitigen Färbung von weißem und grauem Haar

Ein zu etwa 75 Prozent ergrautes, weißes Haar, welches einen deutlichen Gelbschimmer besitzt, wird gewaschen unt mit einem Handtuch frottiert. Anschließend wird das Haar mit der Hälfte eines Verformungsmittels der Zusammensetzung

| | |
|---|---|
| 17,3000 g | Ammoniumsulfit, 34prozentige wäßrige Lösung |
| 13,5000 g | Schweflige Säure (wäßrige Lösung mit 5 Gewichtsprozent $SO_2$-Gehalt) |
| 7,5000 g | Imidazolidin-2-on |
| 3,5000 g | Isopropanol |
| 0,5000 g | Octylphenol, mit 20 Mol Ethylenoxid oxethyliert |
| 0,2000 g | Parfümöl |
| 0,0002 g | C.I. Acid Red 92 (C.I. 45 410) |
| 57,4998 g | Wasser |
| 100,0000 g | |

und einem pH-Wert von 6,7 vorgefeuchtet und auf Wickler (Durchmesser 5 bis 7 Millimeter für eine Dauerwellung und 15 bis 35 Millimeter für eine Haarentkräuselung) gewickelt. Danach wird die restliche Verformungsflüssigkeit auf das gewickelte Haar verteilt. Nach einer zehnminütigen Einwirkungszeit unter zusätzlicher Wärmeeinwirkung wird das Verformungsergebnis kontrolliert und die Verformungsbehandlung gegebenenfalls um 5 Minuten verlängert.

Das Haar wird sodann mit warmem Wasser gründlich gespült und anschließend mit einem Fixiermittel der folgenden Zusammensetzung

| | |
|---|---|
| 4,000 g | Hydrogenperoxid, 50prozentige wäßrige Lösung |
| 1,000 g | Natriumlaurylsulfat |
| 0,600 g | Orthophosphorsäure, 85prozentige wäßrige Lösung |
| 0,200 g | Ethylendiamintetraessigsäure |
| 0,200 g | Parfümöl |
| 0,050 g | Hippursäure |
| 0,004 g | C.I. Solvent Violet 13 (C.I. 60 725) |
| 93,946 g | Wasser |
| 100,000 g | |

behandelt. Der pH-Wert des Fixiermittels beträgt 2,3.

Man verfährt weiter wie in Beispiel 1 beschrieben und erhält ein ausgezeichnet verformtes Haar, dessen Weißanteil eine reine weiße Färbung besitzt, die frei von unerwünschten Gelbtönen ist.

Beispiel 3 Verfahren zur Dauerwellung und gleichzeitigen Färbung von weißem Haar

Weißes, glattes Haar wird wie üblich gewaschen und frottiert. Das Haar wird auf Wickler (Durchmesser 5 bis 10 Millimeter) gewickelt und anschließend mit einem aus einer Mischung der beiden Komponenten A und B bestehenden Dauerwellmittel mehrmals gut durchfeuchtet.

**A.**

| | |
|---|---|
| 5,0000 g | Harnstoff |
| 0,5000 g | Cetylstearylalkohol |
| 0,6000 g | Stearylalkohol, mit 10 Mol Ethylenoxyd oxethyliert |
| 0,1000 g | Natriumlaurylsulfat |
| 0,5000 g | Ammoniumdihydrogenphosphat |
| 2,0000 g | Glycerindiacetat |
| 0,3000 g | Parfümöl |
| 0,0001 g | C.I. Basic Violet 10 (C.I. 45 170) |
| 90,9999 g | Wasser |
| 100,0000 g | |

**B.**

| | |
|---|---|
| 16,0000 g | Monothioglykolsäureglycerinester |

Der pH-Wert der gebrauchsfertigen Mischung von A und B beträgt 5,3.

Das gewickelte Haar wird mit einer Plastikhaube abgedeckt und 15 Minuten mit einem Wärmestrahler erwärmt.

Anschließend wird das Haar mit warmem Wasser gespült und mit einem Fixiermittel vom pH 2,6 und der folgenden Zusammensetzung

| | |
|---|---|
| 5,0000 g | Hydrogenperoxid, 50prozentige wäßrige Lösung |
| 0,5000 g | Octylphenol, mit 20 Mol Ethylenoxid oxethyliert |
| 0,2000 g | Orthophosphorsäure, 85prozentige wäßrige Lösung |
| 0,0500 g | Acetanilid |
| 0,2000 g | Parfümöl |
| 0,0002 g | C.I. Acid Violet 43 (C.I. 60 730) |
| 94,0498 g | Wasser |
| 100,0000 g | |

behandelt, indem man 60 g des Fixiermittels gleichmäßig auf das gewickelte Haar aufträgt, nach 7 Minuten die Wickler entfernt und das abgewickelte Haar mit den restlichen 40 g des Fixiermittels 3 Minuten lang nachbehandelt. Das Haar wird anschließend mit Wasser gespült, zur Wasserwelle gelegt und getrocknet.

Das so erhaltene dauergewellte Haar besitzt eine beständige, klare, leuchtend weiße Farbe.

Beispiel 4 Verfahren zur Entkräuselung und gleichzeitigen Färbung von weißem und grauem Haar

Eine alkalische (pH= 9,6) Haarentkräuselungscreme der folgenden Zusammensetzung

| | |
|---|---|
| 18,7000 g | Ammoniumthiolacetat, 50prozentige wäßrige Lösung |
| 4,2000 g | Ammoniak, 25prozentige wäßrige Lösung |
| 3,0000 g | Stearylalkohol |
| 3,2000 g | Stearylalkohol, mit 20 Mol Ethylenoxid oxethyliert |
| 1,6000 g | Vaseline |
| 0,4000 g | Parfümöl |
| 2,0000 g | Harnstoff |
| 0,0003 g | C.I. Acid Violet 9 (C.I. 45 190) |
| 0,0002 g | C.I. Acid Red 92 (C.I. 45 410) |
| 66,8995 g | Wasser |
| 100,0000 g | |

wird auf weißes (10 Prozent Grauanteil), stark gekraustes Haar partienweise gleichmäßig aufgetragen. Während der Einwirkungszeit des Entkräuselungsmittels (10 bis 15 Minuten) wird das Haar mehrmals glattgekämmt.

Anschließend wird mit warmem Wasser gespült und mit einer unmittelbar vor dem Gebrauch hergestellten Lösung von

| | |
|---|---|
| 9,79 g | Natriumbromat |
| 0,20 g | Natriumlaurylsulfat |
| 0,01 g | C.I. Solvent Violet 13 (C.I. 60 725) |
| 10,00 g | |

in 90 Milliltern warmem Wasser oxidativ fixiert. Der pH-Wert dieses Fixiermittels beträgt 6,9.

Nach einer Einwirkungszeit von 10 Minuten das Haar mit Wasser gespült und anschließend trockengeföhnt.

Das Haar wird auf diese Weise geglättet und gleichzeitig wird der störende Gelbton dauerhaft entfernt.

Alle in der vorliegenden Anmeldung enthaltenen Prozentangaben stellen Gewichtsprozente dar und alle im obigen verwendeten Markenzeichen werden als solche anerkannt.

**Patentansprüche**

1. Verfahren zum gleichzeitigen Färben und dauerhaften Verformen von weißem und grauem Haar, bei dem man das Haar zuerst mit einem Verformungsmittel behandelt, mit Wasser spült, und sodann mit einem Fixiermittel behandelt, dadurch gekennzeichnet, daß man ein Verformungsmittel mit einem Gahalt an mindestens einem rotviolettfärbenden Xanthenfarbstoff und ein Fixiermittel mit einem Gehalt an mindestens einem blau-färbenden Anthrachinonfarbstoff verwendet.

2. Verfahren nach Anspruch 1, dadurch gekennzeichnet, daß man den Farbstoff in dem Verformungsmittel und dem Fixiermittel jeweils in einer Menge von 0,00002 bis 0,012 Gewichtsprozent verwendet.

3. Verfahren nach Anspruch 1 und 2, dadurch gekennzeichnet, daß der Xanthenfarbstoff ausgewählt ist aus Farbstoffen mit der Colour-Index-Number C. I. 45 170, C. I. 45 190 und C. I. 45 410.

4. Verfahren nach Anspruch 1 bis 3, dadurch gekennzeichnet, daß der Anthrachinonfarbstoff ausgewählt ist aus Farbstoffen mit der Colour-Index-Number C. I. 60 725, C. I. 60 730 und C. I. 69 825.

5. Verfahren nach Anspruch 1 bis 4, dadurch gekennzeichnet, daß man als Verformungsmittel eine wäßrige Zubereitung auf der Basis eines haarkeratinreduzierenden Stoffes verwendet.

6. Verfahren nach Anspruch 5, dadurch gekennzeichnet, daß der haarkeratinreduzierende Stoff ein Salz der schwefeligen Säure oder ein Salz oder Ester einer Mercaptocarbonsäure ist.

7. Verfahren nach Anspruch 5 und 6, dadurch gekennzeichnet, daß man den haarkeratinreduzierenden Stoff in dem Verformungsmittel in einer Menge von 2 bis 14 Gewichtsprozent verwendet.

8. Verfahren nach Anspruch 1 bis 7, dadurch gekennzeichnet, daß man dem Verformungsmittel mindestens einen Quell- und Penetrationsstoff zusetzt.

9. Verfahren nach Anspruch 8, dadurch gekennzeichnet, daß der Quell- und Penetrationsstoff ausgewählt ist aus Harnstoff, Melamin, Alkali- oder Ammoniumthiocyanat, Isopropanol, Imidazolidin-2-on, 2-Pyrrolidon und 1-Methyl-2-pyrrolidon.

10. Verfahren nach Anspruch 1 bis 9, dadurch gekennzeichnet, daß man als Fixiermittel eine wäßrige Zubereitung mit einem Gehalt an einem Oxidationsmittel verwendet.

11. Verfahren nach Anspruch 10, dadurch gekennzeichnet, daß das Oxidationsmittel ausgewählt ist aus Hydrogenperoxid, Natrium- oder Kaliumbromat, Natriumperborat und Harnstoffperoxid.

**Claims**

1. Process for the simultaneous dyeing and permanent shaping of white or grey hair, in which the hair is first treated with a shaping composition, then rinsed with water and treated with a fixing composition, characterized in that a shaping composition is used which comprises a content of at least one red-violet dyeing xanthene dyestuff and a fixing composition with a content of at least one blue dyeing anthraquinone dyestuff.

2. Process according to Claim 1, characterized in that the dyestuff is employed in the shaping composition and the fixing composition in a quantity of 0.00002 to 0.012% by weight in each instance.

3. Process according to Claims 1 and 2, characterized in that the xanthene dyestuff is selected from the dyestuffs with the Colour Index numbers C.I. 45 170, C.I. 45 190 and C.I. 45 410.

4. Process according to Claims 1 to 3, characterized in that the anthraquinone dyestuff is selected from the dyestuffs with the Colour Index numbers C.I. 60 725, C.I. 60 730 and C.I. 69 825.

5. Process according to Claims 1 to 4, characterized in that an aqueous preparation based on a hair keratin reducing material is used as shaping composition.

6. Process according to Claim 5, characterized in that the hair keratin reducing material is a salt of sulfuric acid or a salt or ester of a mercaptocarboxylic acid.

7. Process according to Claims 5 and 6, characterized in that the hair keratin reducing material is employed in the shaping composition in a quantity of 2 to 14% by weight.

8. Process according to Claims 1 to 7, characterized in that at least one swelling and penetrating agent is added to the shaping composition.

9. Process according to Claim 8, characterized in that the swelling and penetrating agent is selected from urea, melamine, alkali- or ammonium thiocyanate, isopropanol, imidazolidin-2-one, 2-pyrrolidone and 1-methyl-2-pyrrolidone.

10. Process according to Claims 1 to 9, characterized in that an aqueous preparation with a content of an oxidizing agent is used as fixing composition.

11. Process according to Claim 10, characterized in that the oxidizing agent is selected from hydrogen peroxide, sodium or potassium bromate, sodium perborate and urea peroxide.

**Revendications**

1. Procédé pour simultanément colorer et former de façon durable des cheveux blancs et gris, dans lequel on traite d'abord les cheveux avec un moyen de formage, on les rince à l'eau et on les traite ensuite avec un fixatif, caractérisé en ce qu'on utilise un moyen de formage ayant une teneur en au moins un colorant à base de xanthène donnant une couleur rouge-violet et un fixatif ayant une teneur en au moins un colorant à base d'anthraquinone donnant une couleur bleue.

2. Procédé selon la revendication 1, caractérisé en ce qu'on utilise le colorant dans le moyen de formage et dans le fixatif respectivement selon une quantité de 0,00002 à 0,012% en poids.

3. Procédé selon les revendications 1 et 2, caractérisé en ce que le colorant à base de xanthène est choisi parmi des colorants ayant un indice de couleur I.C. 45 170, I.C. 45 190 et I.C. 45 410.

4. Procédé selon les revendications 1 à 3, caractérisé en ce que le colorant à base d'anthraquinone est choisi parmi des colorants ayant l'indice de couleur I.C. 60 725, I.C. 60 730 et I.C. 69 825.

5. Procédé selon les revendications 1 à 4, caractérisé en ce qu'on utilise comme moyen de formage une préparation aqueuse à base d'une substance réduisant la kératine du cheveu.

6. Procédé selon la revendication 5, caractérisé en ce que la substance réduisant la kératine du cheveu est un sel de l'acide sulfureux, ou un sel ou un ester d'un acide mercaptocarboxylique.

7. Procédé selon la revendication 5 et 6, caractérisé en ce qu'on utilise la substance réduisant la kératine du cheveu dans le moyen de formage selon une quantité comprise entre 2 et 14% en poids.

8. Procédé selon les revendications 1 à 7, caractérisé en ce qu'on ajoute au moyen de formage une substance de gonflement et de pénétration.

9. Procédé selon la revendication 8, caractérisé en ce que la substance de pénétration et de gonflement est choisie parmi l'urée, la mélamine, un thiocyanate d'alcali ou le thiocyanate d'ammonium, l'isopropanol, l'imidazolidine-2-one, le 2-pyrrolidone et le 1-méthyl-2-pyrrolidone.

10. Procédé selon les revendications 1 à 9, caractérisé en ce qu'on utilise comme fixatif une préparation aqueuse contenant un oxydant.

11. Procédé selon la revendication 10, caractérisé en ce que l'oxydant est chosi parmi l'eau oxygénée, le bromate de sodium ou de potassium, le perborate de sodium et le peroxyde d'urée.